# EUROPEAN PATENT APPLICATION

(11) **EP 2 397 073 A2**
(43) Date of publication of application: **21.12.2011**
(21) Application number: 09840106.0
(22) Date of filing: 23.12.2009
(51) Int. Cl.: A61B 5/0476, B60K 28/02, B60K 28/06

(54) **ENGINE DRIVER RECOGNITION SYSTEM AND METHOD USING BRAIN WAVES**

(30) Priority: 10.02.2009 KR 20090010737
(71) Applicant: Korea Railroad Research Institute, Gyeonggi-do 437-757 (KR)
(72) Inventor: SONG, Yong-Soo, Uiwang-si Gyeonggi-do 437-770 (KR); HAN, Seong-Ho, Yongin-city Gyeonggi-do 448-150 (KR); LEE, Myeong-Ho, Seoul 120-749 (KR); CHOI, Sung-Kyou, Seoul 134-061 (KR)
(74) Representative: Petraz, Gilberto Luigi
(86) International application number: PCT/KR2009/007742
(87) International publication number: WO 2010/093116

(57) **Abstract**

The present invention relates to an engine driver recognition system and method using brain waves. In particular, the engine driver recognition system comprises a brain wave detection means for detecting a brain wave signal from a driver of a train in operation; and a data processing means for collecting brain wave signals from the brain wave detection means and analyzing the conscious state of the engine driver, wherein said system detects brain waves of the engine driver in the train, analyzes brain wave signals detected for analysis of the conscious state of the engine driver and then displays analysis results. According to the present invention, monitoring brain waves of an engine driver allows a control center to efficiently decide whether the engine driver has a decreased conscious level in view of alertness, such as, for example, whether the engine driver has fallen asleep, is under anesthetic or feels drowsy. Therefore, when the engine driver is in such a low alert state, the control center can take quick action to cope with any incident.

## Description

### Technical Field

The present invention relates, in general, to a train engine driver recognition system and method using brain waves and, more particularly, to a train engine driver recognition system and method using brain waves, which measure the brain waves of a train engine driver and analyze the brain waves, thus estimating the state of consciousness of the train engine driver.

### Background Art

Generally, in the case of trains used as a public transportation, the role of the engine driver who monitors and controls the operation of the train is very important.

Therefore, such an engine driver must monitor and control the state of a train by demonstrating a strong power of concentration as concerns the operation of the train.

Meanwhile, a train is long-distance transportation, so that an engine driver must control the operation of the train for a long period of time. Accordingly, when the engine driver enters a decreased state of consciousness, such as a sleep state, an anesthetic state, or a drowsy state, he or she copes slowly with an emergency, which occasionally becomes a factor causing a large-scale accident.

In spite of such a problem, a method capable of detecting the state of consciousness of a train engine driver has not yet been presented, so that there is a problem in that a control center which monitors the overall operation of a train cannot check the state of consciousness of the engine driver, thus making it impossible to promptly cope with the state of the engine driver even when the engine driver falls asleep, is under an anesthetic, or feels drowsy.

### Disclosure

### Technical Problem

Accordingly, the present invention has been made keeping in mind the above problems, and an object of the present invention is to provide a train engine driver recognition system and method using brain waves, which can detect the state of consciousness of a train engine driver so as to promptly cope with the state of the train engine driver when the train engine driver enters a decreased state of consciousness, such as a sleep state, an anesthetic state, or a drowsy state.

Another object of the present invention is to provide a train engine driver recognition system and method using brain waves, which can always monitor the state of consciousness of the engine driver of a train by analyzing the brain waves of the engine driver, and can then utilize the monitored results as data required to cause the train to safely operate.

### Technical Solution

In order to accomplish the above objects, the present invention provides a train engine driver recognition system using brain waves, including brain wave measurement means for measuring brain wave signals from an engine driver of a train in operation; and data processing means for collecting the brain wave signals from the brain wave measurement means, and then analyzing a state of consciousness of the engine driver.

In this case, the brain wave measurement means may include a brain wave measurement unit for measuring the brain wave signals of the engine driver, an Analog/Digital (A/D) conversion unit for converting analog brain wave signals measured by the brain wave measurement unit into digital brain wave signals, a signal transmission unit for transmitting the brain wave signals to the data processing means, and a first control unit for monitoring the brain wave measurement unit, and controlling transmission of the brain wave signals by the signal transmission unit.

In particular, the brain wave measurement unit may be implemented as an electrode.

Further, the data processing means may include a signal reception unit for receiving the brain wave signals transmitted from the brain wave measurement means, a memory unit for storing the brain wave signals received via the signal reception unit, and an analysis program for analyzing the brain wave signals, a second control unit for analyzing the brain wave signals received via the signal reception unit, and a display unit for displaying data about the brain wave signals analyzed by the second control unit.

In this case, the train engine driver recognition system may further include a key input unit for inputting conditions required to analyze the brain wave signals.

Further, the data processing means may be either a Personal Computer (PC) or a notebook computer.

Meanwhile, the brain wave measurement means may include a signal transmission unit for transmitting the brain wave signals, and the data processing means may include a signal reception unit for receiving the brain wave signals transmitted from the signal transmission unit, and wherein the signal transmission unit and the signal reception unit are implemented as a transmission/reception module that enables long-range wireless communication.

In addition, the present invention provides a train engine driver recognition method using brain waves, including a first step of measuring brain wave signals of an engine driver of a train using brain wave measurement means; a second step of receiving the brain wave signals measured by the brain wave measurement means, analyzing the brain wave signals, and then analyzing a state of consciousness of the engine driver of the train; and a third step of displaying the analyzed data.

In this case, the brain wave signals acquired by the brain wave measurement means may be low-power frequency signals.

### Advantageous Effects

According to the present invention, there are advantages in that the brain waves of a train engine driver are monitored, and a control center or the like can effectively determine whether a decreased state of consciousness of the engine driver has occurred in an awake state, for example, when falling asleep, being under an anesthetic or feeling drowsy, thus allowing action to be promptly taken to cope with the decreased state of consciousness if the engine driver enters the decreased state of consciousness.

In particular, when action is taken to promptly cope with the operation of a train by measuring the brain waves of a train engine driver in this way, accidents or the like can be prevented.

### Description of Drawings

Fig. 1 is a diagram showing the configuration of normal brain waves; and
Fig. 2 is a diagram showing the construction of a train engine driver recognition system using brain waves according to the present invention.

### Best Mode

The features of a train engine driver recognition system and method using brain waves according to the present invention will be more clearly understood from embodiments which will be described in detail with reference to the accompanying drawings.

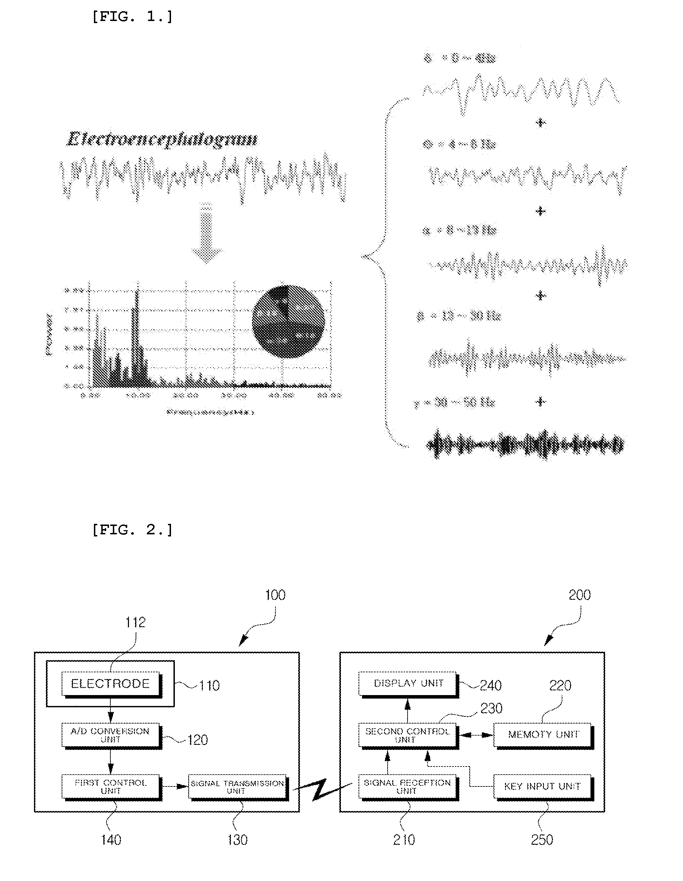

Fig. 1 is a diagram showing the configuration of normal brain waves and Fig. 2 is a diagram showing the construction of a train engine driver recognition system using brain waves according to the present invention.

Referring to Fig. 1, the train engine driver recognition system using brain waves according to the present invention measures brain waves (electroencephalogram: EEG), which correspond to one of various types of biometric signals, from an engine driver on board a train, and analyzes the brain waves (EEG), thus estimating the state of consciousness of the engine driver.

In this case, brain waves (EEG) refer to weak electrical signals of a microvolt level (µV) which are continuously and spontaneously being generated from a living brain and are oscillating 50 or fewer times per second. Those brain waves are composed of various oscillating components which appear as the result of a complicated interaction between excitatory neurons and inhibitory neurons constituting the brain.

Among these components, components oscillating very slow at frequencies of 0 to 4 Hz are called delta waves, components oscillating at frequencies of 4 to 8 Hz are called theta waves, components oscillating at frequencies of 8 to 13 Hz are the natural rhythm of brain waves called alpha waves, and components belonging to relatively fast oscillating components, that is, components oscillating at frequencies of 13 to 30 Hz, are called beta waves and components oscillating at frequencies of 30 to 50 Hz are called gamma waves.

Generally, it is well known that delta and theta waves become prominent in the state in which consciousness is decreased as in the case of a sleep state, an anesthetic state, or a drowsy state, alpha waves become prominent in a stable state, slow beta waves become prominent in a state of concentration, fast beta waves become prominent in an emotionally instable state and an awake state, and gamma waves become prominent in high-level cognitive operations such as complicated computations, reasoning, or determination.

Therefore, when delta waves or theta waves are used, the degree of concentration and the drowsiness of the train engine driver can be perceived.

In this case, in order to grasp the state of consciousness of the train engine driver, both background brain waves (background EEG) in the stable state in which the eyes of the engine driver are closed, and active brain waves (active EEG) in the operating state in which the eyes of the engine driver are open, must be examined. The background EEG is measured as if the engine driver has meditated during a driver changing time or during breaktime, and the active EEG is measured in the engine room of the train. That is, the background EEG is stored as a set value, and variations in the active EEG relative to the background EEG are compared, thus enabling the state of consciousness of the engine driver to be analyzed.

Meanwhile, the term "normal reference range" refers to a standardized range for a normative database. The reference range of biometric indices is generally prescribed to acquire a normal distribution of relevant indices from a large-scale normal person group of several hundreds to several thousands of persons, and take a range spreading to the ends of both intervals, each spaced apart from a mean value located in the middle of the normal distribution by the magnitude of a Standard Deviation (SD), that is, a range from -1SD to +1SD.

In this case, when a standardization procedure is assumed to be a procedure in which individual reference ranges are consistently adjusted to a uniform range so that various biometric indices can be more easily analyzed, it is preferable to use a range having 0-mean and 1-standard deviation (reference range: 1 ∼ 1,Z-score) for academic research purposes, and to use numerical values standardized using an integer level numerical range that allows a patient to be more conveniently examined, that is, a range having 50-mean and 10-standard deviation (reference range: 40 ∼ 60) or a range having 100-mean and 10-standard deviation (reference range: 90 ∼ 110) for clinical utilization purposes.

Therefore, standardized numerical values show an exact statistical reference which enables the objective determination of whether the analyzed EEG index values are higher or lower than those of a normal group, thus enabling an abnormal EEG index to be detected when brain waves (EEG) are examined. Accordingly, the measured EEG signals are compared to the standardized EEG values, so that the EEG signals of the engine driver currently being measured are compared and analyzed, thus enabling the state of consciousness of the engine driver to be determined.

The above-described train engine driver recognition system using brain waves according to the present invention includes a brain wave (EEG) measurement means 100 for measuring the EEG of a train engine driver, and a data processing means 200 for collecting EEG signals from the brain wave measurement means 100 and then analyzing the state of consciousness of the train engine driver, as shown in Fig. 2.

The brain wave measurement means 100 includes a brain wave measurement unit 110 for measuring the EEG of the train engine driver, an Analog/Digital (A/D) conversion unit 120 for converting analog EEG signals measured by the brain wave measurement unit 110 into digital EEG signals, a signal transmission unit 130 for transmitting the digital EEG signals to the data processing means 200, and a first control unit 140 for monitoring the brain wave measurement unit 110 and controlling the transmission of the EEG signals by the signal transmission unit 130.

Meanwhile, the data processing means 200 includes a signal reception unit 210 for receiving the digital EEG signals transmitted from the signal transmission unit 130 of the brain wave measurement means 100, a memory unit 220 for storing the EEG signals received via the signal reception unit 210 and an analysis program, a second control unit 230 for analyzing the EEG signals received via the signal reception unit 210 so as to estimate the state of consciousness of the train engine driver, a display unit 240 for displaying analysis result data about the EEG signals analyzed by the second control unit 230, and a key input unit 250 for inputting conditions required to analyze the EEG signals.

In this case, it is apparent that the data processing means 200 may be implemented using a Personal Computer (PC) or a notebook computer.

Further, the signal transmission unit 130 and the signal reception unit 210 are implemented as a well-known transmission/reception module enabling long-range wireless communication, and thus it is preferable to install and operate the brain wave measurement means 100 within the train and to install and operate the data processing means 200 in a control center located in a remote place.

Meanwhile, the brain wave measurement means 100 measures the EEG signals of the train engine driver. The brain wave (EEG) signals, which are biometric signals that can be measured from the body of the engine driver and can be used as factors for measuring riding comfort on the train, are obtained by recording minute electrical variations in the form of curves, and become important primary factors enabling the state of consciousness of the train engine driver, such as a sleep state, an anesthetic state, or a drowsy state, to be detected.

Such EEG signals are measured by the brain wave measurement unit 110 of the brain wave measurement means 100. For this function, the brain wave measurement unit 110 is implemented as an electrode 112, and measures EEG signals in the form of micro-current flowing through the skin.

Hereinafter, a train engine driver recognition procedure using brain waves according to the present invention will be described in detail with reference to Figs. 1 and 2.

The train engine driver recognition method using brain waves mainly includes the first step of measuring the brain waves (EEG) of a train engine driver using the brain wave measurement means, the second step of receiving EEG signals measured by the brain wave measurement means, analyzing the EEG signals, and then analyzing the state of consciousness of the train engine driver, and the third step of displaying analysis result data.

In this case, the brain wave measurement means 100 is installed in the train, and the EEG signals measured by the brain wave measurement means 100 are transmitted to and processed by the data processing means 200 provided in the control center in the remote place.

Meanwhile, the EEG signals measured by the brain wave measurement means 100 are converted into digital EEG signals via the A/D conversion unit 120, and the digital EEG signals are transmitted via the signal transmission unit 130 under the control of the first control unit 140.

Meanwhile, the digital EEG signals transmitted via the signal transmission unit 130 are received by the signal reception unit 210 of the data processing means 200, and are then analyzed using the analysis program.

Further, the result data analyzed in this way is displayed on the display unit 240 and the state of the EEG signals can be verified.

In this case, whether the EEG signals received via the data processing means 200 correspond to information falling within low-power frequency ranges is determined based on spectrum data about the received EEG signals. The EEG signal data which is source signal data can be adjusted so that the variables of adjusted EEG signal data for acquiring information falling within the low-power frequency ranges are determined by increasing the dynamic range for power falling within the low-power frequency ranges compared to higher-power frequency ranges. That is, the low-power frequency range information can be used to analyze various types of EEG signal data. For example, the sleep state can be determined by using low-power frequency range information which is included in the EEG signal data of the train engine driver, from sleep conducted for a specific period, and the EEG signal analysis of an automated full frequency spectrum can be used for customized analysis such as by evaluating the quality of sleep, detecting a pathological state, and determining the effect related to whether an alarm is given in the sleep state.

Here, there are many cases where the low-frequency range of EEG signals has the maximum energy, and one reason for causing power to increase in these lower frequencies is the low pass characteristic of a skull.

Therefore, EEG signals acquired by the brain wave measurement means 100 are low-power frequency signals and follow a 1/f distribution, but the power of these EEG signals is in inverse proportion to the frequency.

Meanwhile, the EEG signals have typically been examined in time in series increments called epochs.

For example, when each EEG signal is used to analyze sleep, sleep can be segmented into one or more epochs to be used for analysis. The epochs can be segmented into a plurality of sections using a scanning window, wherein the scanning window defines different sections of the time series increment. The scanning window can move via a sliding window, and the sliding window has overlapping time series sequences.

Therefore, the data processing means 200 can analyze various types of sleep states or awake states.

In this case, such a sleep state is described as any distinguishable sleep or wakefulness which is indicative of behavioral characteristics, physical characteristics or signal characteristics. Sleep states include Slow-Wave Sleep (SWS), Rapid Eye Movement sleep (REM), intermediate sleep also called inter or IS state, and an awake state, depending on the states thereof.

Meanwhile, the awake state may substantially be part of the sleep state and may be specified by vigilance which is the level of attentiveness or alertness. The intermediate sleep is **characterized in that** it has intermediate-1 sleep and intermediate-2 sleep.

Further, an artifact may occur during the acquisition of EEG signals, and is data misrepresenting the EEG signals. For example, the internal motion of a user for whom an EEG was recorded may be an artifact, and an example of the artifact includes muscle twitches or the like.

As described above, when the analysis of brain wave (EEG) signals of a train engine driver is performed, the sleep state or awake state of the engine driver can be determined. By means of this determination, action can be taken to promptly cope with the state of the engine driver when the engine driver enters the decreased state of consciousness, such as a sleep state, an anesthetic state, or a drowsy state, thus preventing train accidents from occurring.

Although the preferred embodiments of the present invention have been disclosed, the scope of the present invention is not limited to those embodiments, and extends to the embodiments and substantial equivalents thereof, and the present invention can be variously modified and embodied by those skilled in the art, without departing from the spirit of the invention.

### Mode for Invention

### Industrial Applicability

The present invention relates, in general, to a train engine driver recognition system and method using brain waves and, more particularly, to a train engine driver recognition system and method using brain waves, which measure the brain waves of an engine driver and analyze the brain waves, thus estimating the state of consciousness of the engine driver.

## Claims

1. A train engine driver recognition system using brain waves, comprising:
brain wave measurement means for measuring brain wave signals from an engine driver of a train in operation; and
data processing means for collecting the brain wave signals from the brain wave measurement means, and then analyzing a state of consciousness of the engine driver.

2. The train engine driver recognition system according to claim 1, wherein the brain wave measurement means comprises:
a brain wave measurement unit for measuring the brain wave signals of the engine driver;
an Analog/Digital (A/D) conversion unit for converting analog brain wave signals measured by the brain wave measurement unit into digital brain wave signals;
a signal transmission unit for transmitting the brain wave signals to the data processing means; and
a first control unit for monitoring the brain wave measurement unit, and controlling transmission of the brain wave signals by the signal transmission unit.

3. The train engine driver recognition system according to claim 2, wherein the brain wave measurement unit is implemented as an electrode.

4. The train engine driver recognition system according to claim 1, wherein the data processing means comprises:
a signal reception unit for receiving the brain wave signals transmitted from the brain wave measurement means;
a memory unit for storing the brain wave signals received via the signal reception unit, and an analysis program for analyzing the brain wave signals;
a second control unit for analyzing the brain wave signals received via the signal reception unit; and
a display unit for displaying data about the brain wave signals analyzed by the second control unit.

5. The train engine driver recognition system according to claim 4, further comprising a key input unit for inputting conditions required to analyze the brain wave signals.

6. The train engine driver recognition system according to claim 1, wherein the data processing means is either a Personal Computer (PC) or a notebook computer.

7. The train engine driver recognition system according to claim 1, wherein the brain wave measurement means comprises a signal transmission unit for transmitting the brain wave signals, and the data processing means comprises a signal reception unit for receiving the brain wave signals transmitted from the signal transmission unit, and wherein the signal transmission unit and the signal reception unit are implemented as a transmission/reception module that enables long-range wireless communication.

8. A train engine driver recognition method using brain waves, comprising:
a first step of measuring brain wave signals of an engine driver of a train using brain wave measurement means;
a second step of receiving the brain wave signals measured by the brain wave measurement means, analyzing the brain wave signals, and then analyzing a state of consciousness of the engine driver of the train; and
a third step of displaying the analyzed data.

9. The train engine driver recognition method according to claim 8, wherein the brain wave signals acquired by the brain wave measurement means are low-power frequency signals.
